# EUROPEAN PATENT APPLICATION

(11) **EP 2 366 348 A1**
(43) Date of publication of application: **21.09.2011**
(21) Application number: 11250330.5
(22) Date of filing: 17.03.2011
(51) Int. Cl.: A61B 17/34

(54) **Seal assembly for use with an access device**

(30) Priority: 18.03.2010 US 315069 P; 04.02.2011 US 21002
(71) Applicant: Tyco Healthcare Group LP, New Haven, CT 06511 (US)
(72) Inventor: Richard, Paul, Shelton, CT 06484 (US)
(74) Representative: Soames, Candida Jane

(57) **Abstract**

A surgical access device (100) for use during a surgical procedure is disclosed and includes a housing (102), an access member (106) and a seal assembly (200). The access member extends distally from the housing and is dimensioned for positioning within tissue. The seal assembly is disposed in mechanical cooperation at least partially within the housing and comprises an instrument seal (210) and a zero-closure valve (220). The instrument seal comprises an outer portion and a inner portion. The inner portion has an aperture extending therethrough. The instrument seal is configured to maintain a substantially fluid-tight seal with respect to a surgical instrument inserted therethrough. The zero-closure valve is configured to maintain a substantially fluid-tight seal in the absence of a surgical instrument inserted therethrough. The zero-closure valve includes an annular groove (230) disposed along an inner periphery thereof and is configured and dimensioned to accommodate an outer periphery of the instrument seal.

## Description

### CROSS REFERENCE TO RELATED APPLICATION

The present application claims the benefit of and priority to U.S. Provisional Application Serial No. 61/315,069 filed on March 18, 2010, the entire contents of which are incorporated herein by reference.

### BACKGROUND

### Technical Field

The present disclosure relates to seal assemblies for use with a surgical access device. In particular, the disclosure relates to a seal assembly to be used in combination with an access device such that the seal assembly is adapted to accommodate the insertion of surgical instrumentation, while substantially limiting the communication of fluids therethrough.

### Background of Related Art

Laparoscopic procedures are performed in the interior of the abdomen through a small incision, e.g., through narrow endoscopic tubes or cannulas inserted through a small entrance incision in the skin. Minimally invasive procedures are performed elsewhere in the body, e.g., in the chest, and are often generally referred to as "endoscopic" procedures. Minimally invasive or endoscopic procedures generally require that any instrumentation inserted into the body be sealed, i.e. provisions must be made to ensure that fluids, e.g., gases, do not enter or exit the body through the endoscopic incision as, for example, in surgical procedures in which the surgical region is insufflated. Moreover, endoscopic procedures often require the surgeon to act on organs, tissues, and vessels far removed from the incision, thereby requiring that any instruments used in such procedures be relatively long and narrow.

For such procedures, the introduction of a tube into certain anatomical cavities such as the abdominal cavity is usually accomplished by use of an access device, e.g., a system incorporating a trocar and cannula assembly. A cannula assembly is formed of a cannula attached to a cannula housing which generally includes a seal assembly adapted to maintain a seal across the opening of the seal assembly both with and without an instrument inserted therethrough. Since the cannula is in direct communication with the internal portion of the seal assembly, insertion of the cannula into an opening in the patient's body so as to reach the inner abdominal cavity should be adapted to maintain a fluid tight interface between the abdominal cavity and the outside atmosphere.

Since minimally invasive surgical procedures in the abdominal cavity of the body generally require insufflating gases to raise the cavity wall away from vital organs, the procedure is usually initiated by use of a Verres needle through which a gas is introduced into the body cavity. The gas provides a slight pressure which raises the wall surface of the peritoneum away from the vital organs thereby providing an adequate region in which to operate. Thereafter, a trocar assembly which includes a cannula and a trocar or obturator is inserted within the cannula to puncture the peritoneum, i.e. the inner lining of the abdominal cavity wall. The obturator is removed and laparoscopic or endoscopic surgical instruments may then be inserted through the cannula to perform surgery within the abdominal cavity. The cannula may also be utilized for introducing tubes into the body as for drainage purposes, for specimen removal, for diagnostic evaluations, or the like.

In view of the need to maintain the atmospheric integrity of the inner area of the cavity, a seal assembly for a cannula which permits introduction of an obturator and a wide range of surgical instruments and which maintains the atmospheric integrity of the inner area of the cavity is desirable. Generally, in the context of insufflatory, minimally invasive surgical procedures, cannula assemblies include structures that satisfy two sealing requirements. The first requirement is to provide a substantially fluid tight seal when an instrument is not present in the cannula. The second requirement is to provide a substantially fluid tight seal when an instrument is being introduced into or already is present in the cannula.

### SUMMARY

The present disclosure relates to a surgical access device for use during a surgical procedure and includes a housing, an access member and a seal assembly. The access member extends distally from the housing and is dimensioned for positioning within tissue. The seal assembly is disposed in mechanical cooperation at least partially within the housing and comprises an instrument seal and a zero-closure valve. The instrument seal comprises an outer portion and a inner portion. The inner portion has an aperture extending therethrough. The instrument seal is configured to maintain a substantially fluid-tight seal with respect to a surgical instrument inserted therethrough. The zero-closure valve is configured to maintain a substantially fluid-tight seal in the absence of a surgical instrument inserted therethrough. The zero-closure valve includes an annular groove disposed along an inner periphery thereof and is configured and dimensioned to accommodate an outer periphery of the instrument seal.

In an embodiment, the outer portion of the instrument seal may have a rigidity that is greater than a rigidity of the inner portion of the instrument seal. The outer portion of the instrument seal may include a ring formed from a rigid or semi-rigid material, e.g., plastic. The inner portion of the instrument seal may be elastomeric material, and the elastomeric material may be connected to the ring, such as by being overmolded thereon.

The present disclosure also relates to a seal assembly for use with a surgical access device. The seal assembly comprises an instrument seal and a zero-closure valve. The instrument seal comprises an outer portion and a inner portion. The inner portion has an aperture extending therethrough. The instrument seal is configured to maintain a substantially fluid-tight seal with respect to a surgical instrument inserted therethrough. The zero-closure valve is configured to maintain a substantially fluid-tight seal in the absence of a surgical instrument inserted therethrough, and includes an annular groove disposed along an inner periphery thereof. The annular groove is configured and dimensioned to accommodate an outer periphery of the instrument seal.

The present disclosure also relates to a zero-closure valve for use with a surgical access device. The zero-closure valve comprises a body portion and an annular groove. The body portion includes an inner wall and an outer wall. The annular groove is disposed along an inner periphery of the inner wall and is configured and dimensioned to accommodate an outer periphery of an instrument seal. The zero-closure valve is configured to maintain a substantially fluid-tight seal in the absence of a surgical instrument inserted therethrough.

### BRIEF DESCRIPTION OF DRAWINGS

Various embodiments of the presently disclosed seal assembly are disclosed herein with reference to the drawings, wherein:

FIG. 1 is a cross-sectional view of a cannula assembly and a seal assembly in accordance with an embodiment of the present disclosure;

FIG. 2 is a cross-sectional view of the seal assembly of FIG. 1 within the housing of the cannula assembly;

FIG. 3 is a cross-sectional view of a zero-closure valve of the seal assembly of FIGS. 1 and 2;

FIG. 4 is a perspective view of the zero-closure valve of FIGS. 1-3; and

FIG. 5 is a perspective view of an instrument seal of the seal assembly of FIGS. 1 and 2.

### DETAILED DESCRIPTION OF EMBODIMENTS

Embodiments of the presently disclosed seal assembly will now be described in detail with reference to the drawing figures wherein like reference numerals identify similar or identical elements. As used herein and as is traditional, the term "distal" refers to that portion which is farther from the user while the term "proximal" refers to that portion which is closest to the user.

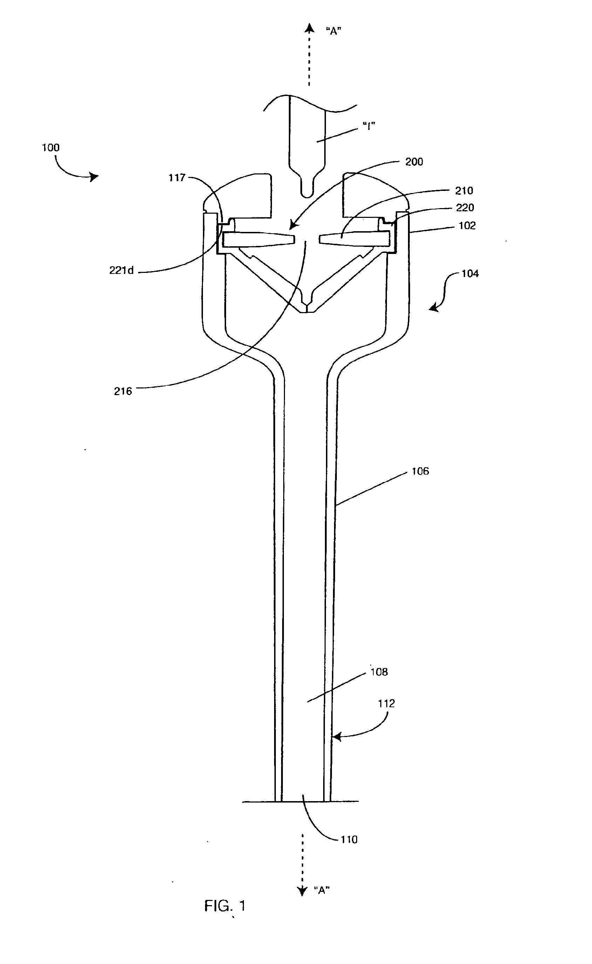

Referring initially to FIG. 1, a surgical access device 100 is illustrated. Access device 100 includes a housing 102 at a proximal end 104 thereof, and an access member 106 extending distally therefrom. Housing 102 is configured and dimensioned to accommodate a seal assembly 200.

Access member 106 is dimensioned for positioning with a percutaneous access point formed in a patient's tissue. Access member 106 defines a lumen 108 extending longitudinally therethrough along a longitudinal axis "A-A." Lumen 108 is configured and dimensioned for the internal receipt of one or more surgical instruments "I." Access member 106 defines an opening 110 at a distal end 112 thereof to allow the surgical instrument(s) "I" to pass therethrough.

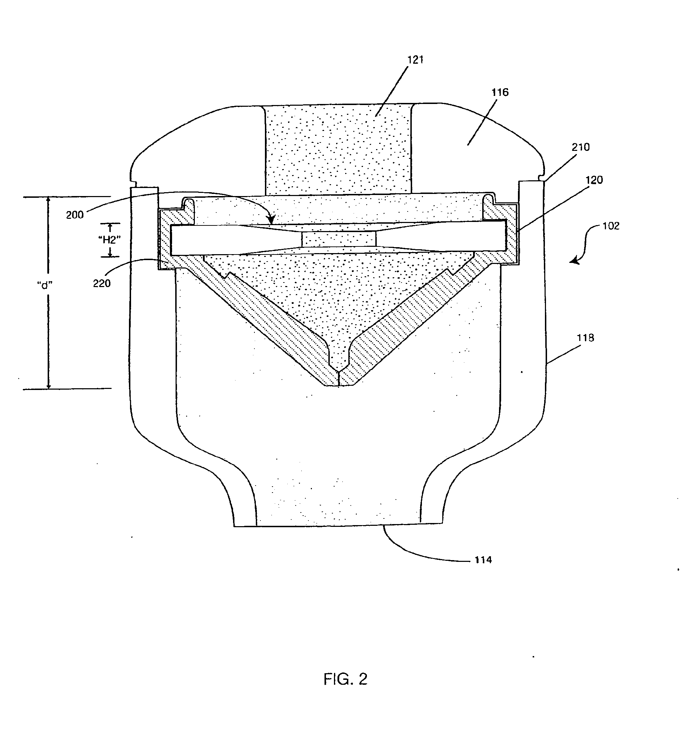

With particular reference to FIG. 2, housing 102 is generally hollow, defining a cavity 114 therein, and is configured for reception of seal assembly 200. Housing 102 also includes a proximal end 116, and side walls 118, which define an annular recess 120. Proximal end 116 of housing 102 may be integrally formed with side walls 118, or may be attachable thereto via suitable means (e.g., a snap-fit connection, frictional fit, etc.). Proximal end 116 includes an opening 121 extending therethrough, such that opening 121 of proximal end 116 is in fluid communication with cavity 114. As shown in FIG. 1, opening 121 is larger than (e.g., has a larger diameter than) a width (e.g., diameter) surgical instrument "I." Accordingly, surgical instrument "I" is insertable through opening 121 of proximal end 116 and into cavity 114 defined within housing 102.

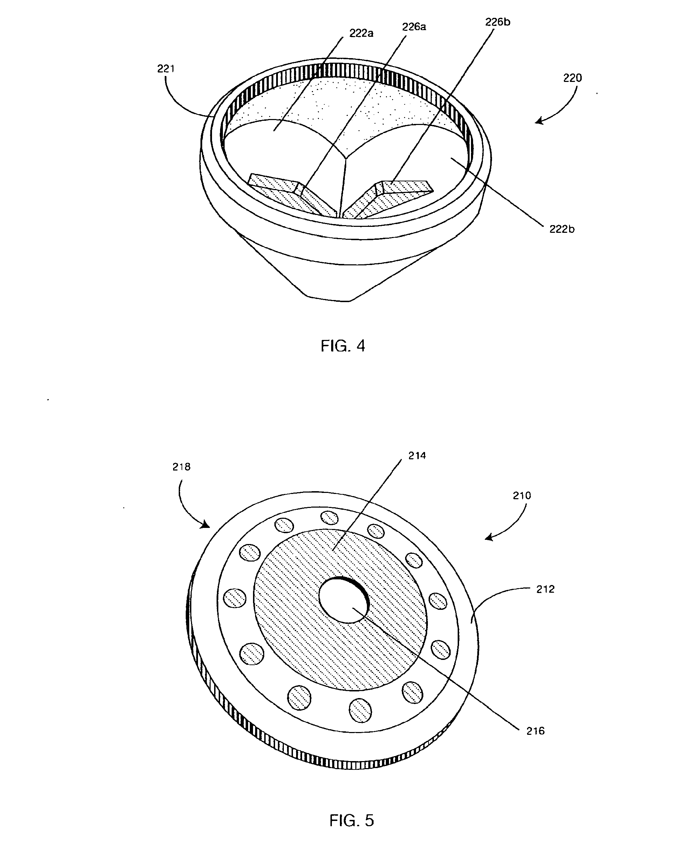

With particular reference to FIGS. 2-5, portions of the seal assembly 200 are illustrated. Seal assembly 200 includes an instrument seal 210 (FIGS. 2 and 5), e.g., a septum valve, and a zero-closure valve 220 (FIGS. 2-4), e.g., a duckbill valve. In the illustrated embodiments, instrument seal 210 includes an outer rigid portion 212 and an inner elastomeric portion 214. The outer rigid portion 212 includes a rigid or semi-rigid ring 213, made from a suitable material such as plastic. The outer rigid portion 212 may derive its rigidity, or semi-rigidity, from the ring 213. In an embodiment, the inner elastomeric portion 214 is connected to the outer rigid portion 212, such as by being, e.g., over-molded onto, the rigid or semi-rigid ring 213. Elastomeric portion 214 includes an aperture 216 extending therethrough, which defines an axis that is substantially parallel to axis "A-A" when instrument seal 210 is in place within housing 102. As illustrated, aperture 216 is smaller than (e.g., has a smaller diameter than) a width (e.g., diameter) of surgical instrument "I" (FIG. 1). In an embodiment, the elastomeric portion 214 is configured to flex (e.g., move generally proximally and/or generally distally) in response to a sufficient amount of force acting thereagainst. The insertion and/or removal of a surgical instrument "I," or the off-axis movement of an instrument "I" therein, may be examples of sufficient amounts of force to cause elastomeric portion 214 to flex or otherwise expand and/or move. Additionally, upon the insertion of a surgical instrument "I" through aperture 216, instrument seal 210 is configured to maintain a substantially fluid-tight seal with respect to the surgical instrument "I." As is commonly used in the field of art, "fluid-tight" is intended to include both "fluid-tight" and "gas-tight."

As shown in FIG. 2, the elastomeric portion 214 may have a thickness H2 at or near its outer periphery while having a relatively smaller thickness at the periphery of the aperture 216. In the embodiment shown, the thickness of the elastomeric portion 214 may taper towards such smaller thickness at the periphery of the aperture 216. Such a tapered thickness of the elastomeric portion 214 may provide the elastomeric portion 214 with improved sealing characteristics while reducing the forces exerted by the elastomeric material on an instrument "I" as the instrument "I" is inserted, removed and/or manipulated within the aperture 216.

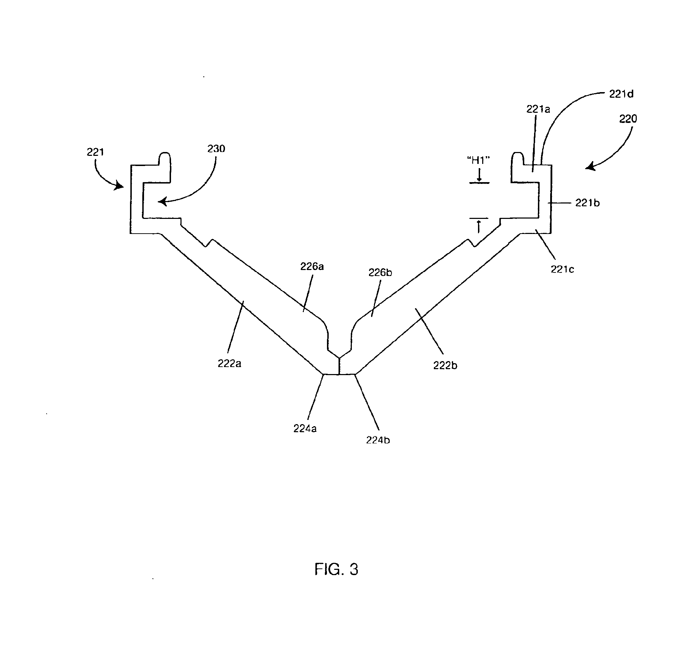

Referring to FIGS. 2-5, zero-closure valve 220 is shown. With particular reference to FIGS. 3 and 4, zero-closure valve 220 includes an outer, annular lip 221 that is configured for engagement with annular recess 120 of housing 102. Zero-closure valve 220 includes a pair of inwardly and distally depending walls 222a, 222b. Distal ends 224a, 224b of each wall 222a, 222b, respectively, extend transversely across at least a portion of cavity 114, and contact one another in a sealing relationship in the absence of surgical instrumentation "I" inserted therebetween. Accordingly, zero-closure valve 220 is configured to prevent the escape of insufflation gases in the absence of surgical instrumentation "I" through housing 102. Each wall 222a, 222b also includes a generally proximally extending protrusion 226a, 226b, respectively, thereon. Protrusions 226a, 226b are configured to receive initial contact from a distal end of surgical instrumentation "I." Protrusions 226a, 226b may be configured as one or more ribs having flat and/or curved upper surfaces that receive initial contact from the distal end of a surgical instrument, or protrusions 226a, 226b may instead be configured as one or more plates having generally flat upper surfaces for receiving such initial contact from the distal end of a surgical instrument.

Zero-closure valve 220 also includes an annular groove 230 disposed along an inner periphery of annular lip 221. Annular groove 230 is configured and dimensioned to accommodate an outer periphery 218 of instrument seal 210 therein, such that zero-closure valve 220 and instrument seal 210 are positionable within housing 102 as a single unit. That is, annular groove 230 includes a height "H1" that is slightly larger than, substantially equal to, or slightly smaller than a height "H2" of instrument seal 210, thus enabling a frictional fit therebetween.

More particularly, annular groove 230 divides annular lip 221 into a proximal portion 221a, an outer portion 221b and a distal portion 221c. At least a portion of zero-closure valve 220 (e.g., annular lip 221) may be made of a material that is capable of flexing. As such, it is envisioned that proximal portion 221 a of annular lip 221 can be flexed (e.g., by hand or an instrument) with respect to outer portion 221b and/or distal portion 221 c to allow instrument seal 210 to be inserted into and removed from annular groove 230. Also, a distally-projecting annular lip (see FIG. 1) engages a proximal face 221d of the proximal portion 221 a so as to press down upon and create a seal between the various sealing surfaces within the valve housing 102. The rigidity, or semi-rigidity, of the ring 213 prevents the instrument seal 216 from being dislodged from the annular groove 230 of the zero-closure valve 220 during operation, e.g., when the insertion, removal or manipulation of an instrument "I" causes the instrument "I" to exert forces thereon.

Therefore, in accordance with the present disclosure, it is envisioned that portions of instrument seal 210 and zero-closure valve 220 are made from suitable materials including but are not limited to elastomeric materials such as natural rubber, synthetic polyisoprene, butyl rubber, halogenated butyl rubbers, polybutadiene, styrene-butadiene rubber, nitrile rubber, hydrogenated nitrile rubbers, chloroprene rubber, ethylene propylene rubber, ethylene propylene diene rubber, epichlorohydrin rubber, polyacrylic rubber, silicone rubber, fluorsilicone rubber, fluoroelastomers, perfluoroelastomers, polyether block amides, chlorosulfonated polyethylene, ethylene-vinyl acetate, thermoplastic elastomers, thermoplastic vulcanizers, thermoplastic polyurethane, thermoplastic olefins, resilin, elastin, and polysulfide rubber.

As can be appreciated, the utilization of both instrument seal 210 and zero-closure valve 220 of the present disclosure results in a seal assembly 200 having a relatively low profile. More particularly, with reference to FIG. 2, the proximal-to-distal distance "d" of seal assembly 200 may be less than the proximal-to-distal distance of a conventional seal assembly. For example, in a conventional seal assembly, the instrument seal and the zero-closure valves are longitudinally spaced part from each other, thereby causing such conventional seal assemblies to take up more space than the seal assembly of the present invention. By reducing the longitudinal dimension of the seal assembly, the housing 102 of the present invention may be better suited for use in surgical procedures where a low-profile valve assembly is desired. Such surgical procedures may include, for example, single incision laparoscopic surgeries or pediatric surgeries, or any surgery where multiple access devices are arranged in close proximity with each other, with other surgical equipment, or with anatomical regions of the patient. When maintained in close proximity to each other, larger conventional valve assemblies may tend to clash into each other, preventing a surgeon from positioning them as desired or otherwise impeding the surgical procedure - in contrast, the smaller, low-profile valve assemblies of the present invention provide a relative decrease in the likelihood of the valve assemblies undesirably clashing with each other or with other objects during a surgical procedure.

As discussed above, zero-closure valve 220 and instrument seal 210 are positionable within housing 102 as a single unit. It is envisioned that positioning seal assembly 200, including zero-closure valve 220 and instrument seal 210, as a single unit takes less time, and is thus less expensive to manufacture and/or assemble than conventional valve assemblies. Further, in typical seal assemblies (wherein the instrument seal and the zero-closure valves are longitudinally spaced apart from each other), each of the zero-closure valve and instrument seal is in contact with an inner wall of the housing, thus creating at least two possible leak sites relative to the inner wall of the housing, e.g., two locations (e.g., between an outer periphery of the zero-closure valve and an inner wall of the housing, and between an outer periphery of the instrument seal and an inner wall of the housing) where fluids can undesirably enter and/or exit the housing. In contrast, during utilization of the disclosed seal assembly 200 of the present disclosure, the number of such possible leak sites is reduced. Specifically, the only possible leak site relative to the inner wall of the housing is the connection region between annular lip 221 of zero-closure valve 220 and inner wall/annular recess 120 of housing 102. In this manner, the disclosed seal assembly 200 of the present disclosure may provide an improved seal performance as compared to conventional seal assemblies.

It will be understood that various modifications may be made to the embodiments of the presently disclosed seal assembly described above. Therefore, the above description should not be construed as limiting, but merely as exemplifications of preferred embodiments. Those skilled in the art will envision other modifications within the scope and spirit of the present disclosure.

The invention may be described by reference to the following numbered paragraphs:

A surgical access device for use during a surgical procedure, the surgical access device comprising: a housing; an access member extending distally from the housing and being dimensioned for positioning within tissue, the housing and the access member defining a longitudinal axis; and a seal assembly disposed in mechanical cooperation at least partially within the housing, the seal assembly comprising: an instrument seal comprising an outer portion and a inner portion, the inner portion having an aperture extending therethrough, the instrument seal being configured to maintain a substantially fluid-tight seal with respect to a surgical instrument inserted therethrough; and a zero-closure valve configured to maintain a substantially fluid-tight seal in the absence of a surgical instrument inserted therethrough, the zero-closure valve including an annular groove disposed along an inner periphery thereof, the annular groove being configured and dimensioned to accommodate an outer periphery of the instrument seal.

The surgical access device of paragraph [0031], wherein the annular groove of the zero-closure valve is formed within a proximally-disposed annular lip of the zero-closure valve.

The surgical access device of paragraph [0031], wherein the annular lip is configured to engage an annular recess of the housing.

The surgical access device of paragraph [0032], wherein the annular lip includes a proximal portion, an outer portion and a distal portion.

The surgical access device of paragraph [0032], wherein at least a portion of the annular lip is made from a flexible material.

The surgical access device of paragraph [0034], wherein the proximal portion of the annular lip is made from a flexible material.

The surgical access device of paragraph [0034], wherein the proximal portion of the annular lip can be flexed with respect to the outer portion of the annular lip.

The surgical access device of paragraph [0031], wherein the instrument seal is insertable into the annular groove of the zero-closure valve, and wherein the instrument seal is removable from the annular groove of the zero-closure valve.

The surgical access device of paragraph [0031], wherein the outer portion of the instrument seal has a rigidity that is greater than a rigidity of the inner portion of the instrument seal.

The surgical access device of paragraph [0031], wherein the outer portion of the instrument seal includes a ring formed from a rigid or semi-rigid material.

The surgical access device of paragraph [0040], wherein the ring is formed of plastic.

The surgical access device of paragraph [0031], wherein the inner portion of the instrument seal is elastomeric material.

The surgical access device of paragraph [0042], wherein the outer portion of the instrument seal includes a ring formed from a rigid or semi-rigid material, and wherein the elastomeric material is connected to the ring.

The surgical access device of paragraph [0043], wherein the elastomeric material is connected to the ring by being overmolded thereon.

A seal assembly for use with a surgical access device, the seal assembly comprising: an instrument seal comprising an outer portion and a inner portion, the inner portion having an aperture extending therethrough, the instrument seal being configured to maintain a substantially fluid-tight seal with respect to a surgical instrument inserted therethrough; and a zero-closure valve configured to maintain a substantially fluid-tight seal in the absence of a surgical instrument inserted therethrough, the zero-closure valve including an annular groove disposed along an inner periphery thereof, the annular groove being configured and dimensioned to accommodate an outer periphery of the instrument seal.

A zero-closure valve for use with a surgical access device, the zero-closure valve comprising: a body portion including an inner wall and an outer wall; and an annular groove disposed along an inner periphery of the inner wall, the annular groove being configured and dimensioned to accommodate an outer periphery of an instrument seal; wherein the zero-closure valve is configured to maintain a substantially fluid-tight seal in the absence of a surgical instrument inserted therethrough.

## Claims

1. A surgical access device for use during a surgical procedure, the surgical access device comprising:
a housing;
an access member extending distally from the housing and being dimensioned for positioning within tissue, the housing and the access member defining a longitudinal axis; and
a seal assembly disposed in mechanical cooperation at least partially within the housing, the seal assembly comprising:
an instrument seal comprising an outer portion and a inner portion, the inner portion having an aperture extending therethrough, the instrument seal being configured to maintain a substantially fluid-tight seal with respect to a surgical instrument inserted therethrough; and
a zero-closure valve configured to maintain a substantially fluid-tight seal in the absence of a surgical instrument inserted therethrough, the zero-closure valve including an annular groove disposed along an inner periphery thereof, the annular groove being configured and dimensioned to accommodate an outer periphery of the instrument seal.

2. The surgical access device of Claim 1, wherein the annular groove of the zero-closure valve is formed within a proximally-disposed annular lip of the zero-closure valve.

3. The surgical access device of Claim 1 or Claim 2, wherein the annular lip is configured to engage an annular recess of the housing.

4. The surgical access device of Claim 2 or Claim 3, wherein the annular lip includes a proximal portion, an outer portion and a distal portion.

5. The surgical access device of any preceding Claim, wherein at least a portion of the annular lip is made from a flexible material.

6. The surgical access device of Claim 4, wherein the proximal portion of the annular lip is made from a flexible material.

7. The surgical access device of any of Claims 4 to 6, wherein the proximal portion of the annular lip can be flexed with respect to the outer portion of the annular lip.

8. The surgical access device of any preceding Claim, wherein the instrument seal is insertable into the annular groove of the zero-closure valve, and wherein the instrument seal is removable from the annular groove of the zero-closure valve.

9. The surgical access device of any preceding Claim, wherein the outer portion of the instrument seal has a rigidity that is greater than a rigidity of the inner portion of the instrument seal.

10. The surgical access device of Claim 9, wherein the outer portion of the instrument seal includes a ring formed from a rigid or semi-rigid material, preferably wherein the ring is formed of plastic.

11. The surgical access device of any preceding Claim, wherein the inner portion of the instrument seal is elastomeric material.

12. The surgical access device of Claim 11, wherein the outer portion of the instrument seal includes a ring formed from a rigid or semi-rigid material, and wherein the elastomeric material is connected to the ring.

13. The surgical access device of Claim 12, wherein the elastomeric material is connected to the ring by being overmolded thereon.

14. A seal assembly for use with a surgical access device, the seal assembly comprising:
an instrument seal comprising an outer portion and a inner portion, the inner portion having an aperture extending therethrough, the instrument seal being configured to maintain a substantially fluid-tight seal with respect to a surgical instrument inserted therethrough; and
a zero-closure valve configured to maintain a substantially fluid-tight seal in the absence of a surgical instrument inserted therethrough, the zero-closure valve including an annular groove disposed along an inner periphery thereof, the annular groove being configured and dimensioned to accommodate an outer periphery of the instrument seal.

15. A zero-closure valve for use with a surgical access device, the zero-closure valve comprising:
a body portion including an inner wall and an outer wall; and
an annular groove disposed along an inner periphery of the inner wall, the annular groove being configured and dimensioned to accommodate an outer periphery of an instrument seal;
wherein the zero-closure valve is configured to maintain a substantially fluid-tight seal in the absence of a surgical instrument inserted therethrough.
